# EUROPÄISCHE PATENTANMELDUNG

(11) **EP 3 981 458 A1**
(43) Veröffentlichungstag der Anmeldung: **13.04.2022**
(21) Anmeldenummer: 20211523.4
(22) Anmeldetag: 03.12.2020
(51) Int. Cl.: A61M 16/10

(54) **BEATMUNGSSYSTEM**

(30) Priorität: 09.10.2020 DE 202020105802 U
(71) Anmelder: Müller, Gerhard, 23812 Wahlstedt (DE)
(72) Erfinder: Müller, Gerhard, 23812 Wahlstedt (DE)
(74) Vertreter: Marschall, Stefan

(57) **Zusammenfassung**

Die Erfindung betrifft ein Beatmungssystem zur Verbesserung der Beatmung eines Patienten, mit einem Beatmungsgerät und mit einer Filtrationseinrichtung zur Reduzierung einer Keimbelastung von Ausatemluft des Patienten. Die Filtrationseinrichtung weist einen Filter und eine Heizung zum Beheizen des Filters auf, wobei die Erwärmung des Filters derart erfolgt, dass ein Kondensationsbereich der Ausatemluft stromabwärts und außerhalb des Filters liegt. Durch die Filtrationseinrichtung des Beatmungssystems eine Kontamination der Außenumgebung mit Keimen des Patienten, wie beispielsweise Viren, Bakterien, Pilzen, zuverlässig vermieden.

## Beschreibung

Die Erfindung betrifft ein Beatmungssystem zur Verbesserung der Beatmung eines Patienten, mit einem Beatmungsgerät und mit einer Filtrationseinrichtung zur Reduzierung einer Keimbelastung von Ausatemluft des Patienten.

Aus dem Stand der Technik sind Beatmungsgeräte für die IPV, das heißt die so genannte "Intrapulmonare Perkussive Ventilation", bekannt. Derartige Beatmungsgeräte kommen zur lungenschonenden Beatmung von Patienten verbreitet zum Einsatz. Als Patienten werden in diesem Zusammenhang Menschen oder auch Tiere verstanden, die auf Grund unterschiedlicher Erkrankungen der Lunge, der Atemwege, der für das Atmen notwendigen Muskulatur oder des Nervensystems, das die Muskulatur ansteuert, nicht ausreichend atmen und daher größere Sekretansammlungen in der Lunge ausbilden.

Im Fall einer Beatmung in halboffenen Beatmungsgeräten, wie zum Beispiel bei einer Heimbeatmung mit Ein- und Doppelschlauchsystemen, ist generell von einer unerwünschten Belastung der Ausatemluft mit Keimen, wie Viren, Bakterien und Pilzen auszugehen. Bei der überwiegenden Anzahl der Patienten wurde das Problem bislang zwar erkannt, allerdings noch nicht als relevant angesehen. Diese Einstellung hat sich jedoch durch die weltweite Pandemie mit Covid-19 grundlegend geändert.

Abhilfe kann grundsätzlich durch die Filtration der von einem Beatmungsgerät emittierten Ausatemluft über handelsübliche Bakterienfilter geschaffen werden. Limitierungen ergeben sich jedoch aufgrund der hohen Feuchtigkeit der Ausatemluft von bis zu 98 % relativer Feuchte und noch wesentlich verstärkt beim Vorhandensein einer aktiven Befeuchtung der von dem Beatmungsgerät dem Patienten zugeführten Einatemluft. Durch die Kondensation der Ausatemluft in den eingesetzten Filtern verändert sich deren Durchflusswiderstand und beeinträchtigt die Therapie bis hin zum vollständigen Therapieversagen. Darüber hinaus können die Filter verkeimen und die Umgebung durch das Freisetzen von Viren, Bakterien und Pilzen gefährden, was unter anderem unter Arbeitsschutzaspekten nicht tolerabel ist.

Eine Aufgabe der Erfindung ist es, ein neuartiges Beatmungssystem für Patienten bereitzustellen, das eine mikrobielle Kontamination der Umgebung vermeidet.

Die eingangs genannte Aufgabe wird dadurch gelöst, dass die Filtrationseinrichtung einen Filter und eine Heizung zum Beheizen des Filters aufweist, wobei die Erwärmung des Filters derart erfolgt, dass ein Kondensationsbereich der Ausatemluft stromabwärts und außerhalb des Filters liegt.

Infolgedessen wird eine Kondensation von in der Ausatemluft des Patienten enthaltener Feuchtigkeit innerhalb der Filtrationseinrichtung und/oder dem eingesetzten Beatmungsgerät und eine damit verbundene Kontamination der Ausatemluft mit Viren, Bakterien und Pilzen zuverlässig verhindert. Mittels des Beatmungssystems ist neben einer IPV-Therapie auch eine CPAP-Therapie möglich. Mittels des erfindungsgemäßen Systems können Mundstücke, Nasenmasken oder Gesichtsmasken Verwendung finden.

Bevorzugt weist die Filtrationseinrichtung eine Regeleinrichtung zum Regeln einer Temperatur stromabwärts des Filters auf. Infolgedessen ist eine zuverlässige Verhinderung der Kondensation möglich. Hierbei sollte eine Temperatur des Filters mindestens 2 °C höher als eine Temperatur der Ausatemtemperatur des Patienten sein. Bevorzugt wird der Filter auf etwa 52 °C aufgeheizt, wobei eine Temperatur der hinter dem Filter austretenden Luft 45 °C nicht übersteigt.

Im Fall einer vorteilhaften Ausgestaltung weist das Beatmungsgerät einen Einatemschlauch und einen Ausatemschlauch auf, die mittels eines Y-Verbindungsstücks zu einem Beatmungsschlauch zur Beatmung des Patienten zusammengeführt sind, wobei die Filtrationseinrichtung patientenfern und außerhalb des Beatmungsgeräts in dem Ausatemschlauch angeordnet ist. Hierdurch ist ein besonders robust aufgebautes Doppelschlauchsystem gegeben, das zur Heimbeatmung eines Patienten geeignet ist.

Bevorzugt weist der Einatemschlauch eine Konditioniereinrichtung auf. Hierdurch ist beispielsweise eine definierte Befeuchtung und/oder Temperierung der dem Patienten zugeführten Einatemluft möglich.

Nach Maßgabe einer günstigen Weiterbildung ist der Ausatemschlauch zumindest abschnittsweise beheizbar. Hierdurch wird eine Kondensation der eine hohe Feuchtigkeit aufweisenden Ausatemluft innerhalb des zumindest bis zur Filtrationseinrichtung führenden Ausatemschlauchs vermieden.

Nach Maßgabe einer Ausführungsform des Beatmungssystems ist die Ausatemluft des Patienten zunächst durch die Filtrationseinrichtung und dann durch das Beatmungsgerät führbar. Hierdurch wird eine Kontamination des Beatmungsgeräts mit Patientenkeimen vermieden.

Gemäß eine weiteren Ausgestaltung des Beatmungssystems ist die Ausatemluft des Patienten zunächst durch das Beatmungsgerät und dann durch die Filtrationseinrichtung führbar. Infolgedessen ist eine weitere Möglichkeit zur räumlichen Anordnung der Filtrationseinrichtung in Bezug zum Beatmungsgerät des Beatmungssystems gegeben.

Gemäß einer weiteren günstigen Ausgestaltung ist das Beatmungsgerät mittels eines Zuführschlauchs mit einem patientennahen Ausatemventil verbunden, wobei ein Auslassanschluss des Ausatemventils direkt oder mittels eines vorzugsweise beheizbaren Ausatemschlauchs mit der Filtrationseinrichtung verbunden ist und der Patient über einen Beatmungsanschluss des Ausatemventils beatembar ist. Hierdurch ist die Filtrationseinrichtung auch im Fall eines Einschlauchsystems zuverlässig vor Kondensation geschützt. Die Ansteuerung des Ausatemventils erfolgt bevorzugt durch das Beatmungsgerät.

Nach Maßgabe einer weiteren vorteilhaften Ausgestaltung ist das Beatmungsgerät mittels eines Zuführschlauchs mit einem patientennahen Ausatemstück verbunden, wobei ein Auslassanschluss des Ausatemstücks mit der Filtrationseinrichtung verbunden ist und der Patient über einen Beatmungsanschluss des Ausatemstücks beatembar ist. Hierdurch ist die Filtrationseinrichtung auch im Fall eines Einschlauchsystems zuverlässig vor Kondensation geschützt.

Bevorzugt weist das Ausatemstück und/oder der Auslassanschluss eine Querschnittsreduzierung auf, derart, dass beim Ausatmen des Patienten ein Ausatemwiderstand entsteht durch den die Ausatemluft bevorzugt zu der Filtrationseinrichtung geleitet wird. Im Gegensatz zu dem aktiv durch das Beatmungsgerät angesteuerten Ausatemventil ermöglicht das rein passiv wirkende, nach Art eines T-Stücks ausgeführte Ausatemstück einen konstruktiv besonders einfachen und robusten Aufbau des Einschlauchsystems.

Im Fall einer günstigen technischen Weiterbildung weist der Zuführschlauch eine Konditioniereinrichtung auf. Hierdurch ist beispielsweise eine definierte Befeuchtung und/oder Temperierung der dem Patienten zugeführten Einatemluft möglich.

Bevorzugt ist die Heizung des Filters der Filtrationseinrichtung mit mindestens einer beheizbaren Kontaktfläche, mindestens einem, den Filter zumindest bereichsweise umschließenden Heizkörper, einem Infrarotstrahler, einem elektrischen Widerstandsheizelement oder dergleichen gebildet und der Regeleinrichtung ist mindestens ein bevorzugt im Bereich des Filters angeordneter Temperatursensor zugeordnet. Hierdurch ist eine besonders zuverlässige, präzise regelbare und effiziente Beheizung des Filters realisierbar. Weiterhin kann neben dem mindestens einen Temperatursensor mindestens ein Feuchtesensor vorgesehen sein, sodass der Filter und gegebenenfalls die beheizbaren Schläuche zur Energieeinsparung in Abhängigkeit vom aktuellen Feuchtegrad der Ausatemluft des Patienten beheizbar sind.

Im Folgenden wird ein bevorzugtes Ausführungsbeispiel der Erfindung anhand von schematischen Figuren näher erläutert. Es zeigt
- Figur 1: eine schematische Darstellung einer ersten Ausführungsform eines Beatmungssystems,
- Figur 2: eine schematische Darstellung einer zweiten Ausführungsform eines Beatmungssystem,
- Figur 3: eine schematische Darstellung einer dritten Ausführungsform eines Beatmungssystems, und
- Figur 4: eine schematische Darstellung einer vierten Ausführungsform eines Beatmungssystems.

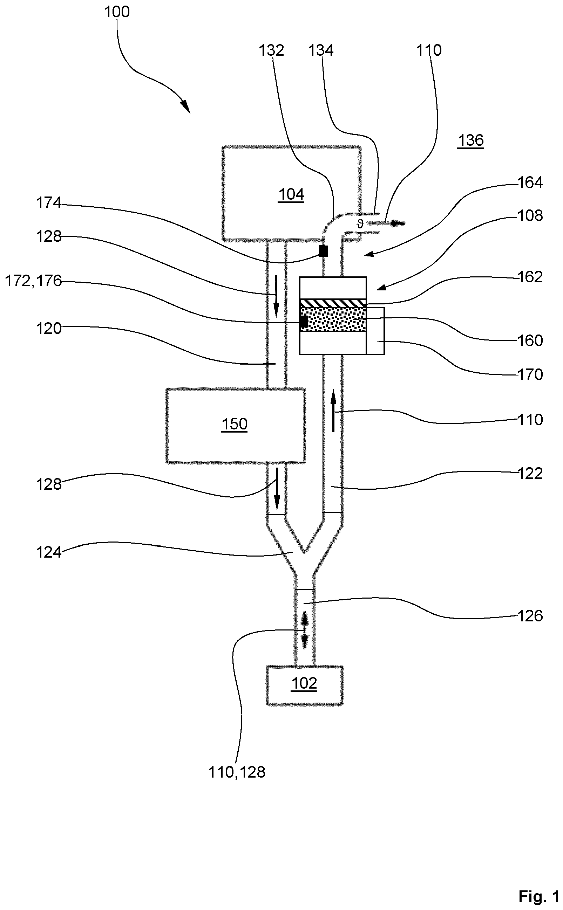

Die Fig. 1 illustriert eine schematische Darstellung einer ersten Ausführungsform eines Beatmungssystems. Ein Beatmungssystem 100 zur Verbesserung der Beatmung eines menschlichen oder tierischen Patienten 102 umfasst unter anderem ein Beatmungsgerät 104 sowie eine Filtrationseinrichtung 108 zur Reduzierung einer Keimbelastung der Ausatemluft 110 des Patienten. Das Beatmungsgerät 104 des Beatmungssystems 100 verfügt über einen Einatemschlauch 120 und einen Ausatemschlauch 122, die mittels eines Y-Verbindungsstücks 124 zu einem Beatmungsschlauch 126 zur Beatmung des Patienten 102 zusammengeführt sind. Mittels des Einatemschlauchs 120 und dem Y-Stück 124 mit dem nachfolgenden Beatmungsschlauch 126 wird dem Patienten 102 von dem Beatmungsgerät 104 bereitgestellte Einatemluft 128 zugeführt und über den Ausatemschlauch 122 als keimbelastete Ausatemluft 110 wieder in Richtung des Beatmungsgeräts 104 abgeführt.

In den Einatemschlauch 120 ist vorzugsweise eine Konditioniereinrichtung 150 eingeschleift, um die Einatemluft 128 für den Patienten 102 auf eine Temperatur von etwa 37 °C bei einer relativen Feuchtigkeit von ungefähr 98 % zu bringen. Innerhalb des Beatmungsschlauchs 126 strömen die Einatemluft 128 und die Ausatemluft 110 in entgegengesetzten Richtungen, was mit einem schwarzen Doppelpfeil veranschaulicht ist. Ein stromabwärts liegender Bogen 132 des Ausatemschlauchs 122 führt hier exemplarisch durch das Beatmungsgerät 104 hindurch bis zu einem Auslass 134 in eine Außenumgebung 136 des Beatmungssystems 100.

Die Filtrationseinrichtung 108 weist hier einen patientenfernen Filter 160 und eine diesem zugeordnete Heizung 162 für die Ausatemluft 110 auf. Die Erwärmung des Filters 160 mittels der Heizung 162 erfolgt hierbei derart, dass ein Kondensationsbereich 164 bzw. eine Kondensationszone - das heißt der räumliche Ort, in dem die in der Ausatemluft 110 des Patienten 102 enthaltene Feuchtigkeit ohne Beheizung kondensieren würde - stromabwärts und außerhalb des Filters 160 sowie des Beatmungsgeräts 104 liegt. Die Kondensation soll möglichst erst in der Außenumgebung 136 erfolgen. Der Begriff "patientenfern" charakterisiert im Kontext der vorliegenden Beschreibung, dass die Filtrationseinrichtung 108 in einen räumlichen Abstand von ca. einem Meter zum Patienten 102 positioniert ist, wohingegen der Begriff "patientennah" entsprechend eine Entfernung von weniger als einem Meter bedeutet.

Der Filter 160 ist dazu ausgebildet, einen möglichst großen Anteil von insbesondere pathogenen mikrobiellen Kontaminationen, beispielsweise in der Form von Bakterien, Viren, Pilzen etc. zurückzuhalten, so dass die Ausatemluft 110 stromabwärts nach dem Filter 160 zwar noch eine hohe relative Feuchtigkeit aufweist, jedoch weitestgehend frei von mikrobiellen Kontaminationen bzw. keimfrei ist. Der Filter 160 kann zu diesem Zweck mehrteilig bzw. mehrlagig aufgebaut sein. Die Filtrationseinrichtung 108 weist ferner eine elektronische Regeleinrichtung 170 auf, mittels der eine Temperatur ϑ stromabwärts des Filters 160 derart regelbar ist, dass keine Kondensation der feuchtigkeitsbeladenen Ausatemluft 110 innerhalb des Kondensationsbereichs 164 auftritt. Zu diesem Zweck wirkt die Regeleinrichtung 170 mit mindestens einem Temperatursensor 172 zusammen, der hier lediglich beispielhaft im Bereich des Filters 160 und der Heizung 162 der Filtrationseinrichtung 108 platziert ist. Mindestens ein weiterer Temperatursensor 174 kann beispielsweise auch weiter stromabwärts hinter der Filtrationseinrichtung 108 im Bereich des Bogens 132 des Ausatemschlauchs 122 oder noch weiter stromabwärts im Bereich des Auslasses 134 des Ausatemschlauchs 122 positioniert sein. Weiterhin kann mindestens ein Feuchtigkeitssensor 176 und/oder mindestens ein Betauungssensor vorgesehen sein, der mit der Regeleinrichtung 170 zusammenwirkt. Darüber hinaus kann zumindest der Ausatemschlauch 122 zumindest abschnittsweise beheizbar ausgeführt sein.

Die mindestens eine Heizung 162 der Filtrationseinrichtung 108 kann mit mindestens einer beheizbaren Kontaktfläche, mindestens einem, den Filter 160 zumindest bereichsweise umschließenden Heizkörper, einem Infrarotstrahler, einem elektrischen Widerstandsheizelement - wie einer Heizfolie, einem Heizdraht etc. - oder dergleichen realisiert sein.

Durch die Heizung 162 wird eine unerwünschte Kondensation der Atemluft 110 des Patienten 102 im Filter 160 sowie stromabwärts hinter dem Filter 160 der Filtrationseinrichtung 108 innerhalb des Kondensationsbereichs 164 zuverlässig vermieden, die ansonsten einen Nährboden für Keime aller Art bilden könnte. Durch den Filter 160 der Filtrationseinrichtung 108 ist zudem die Keimfreiheit der Ausatemluft 110 des Patienten, die letztlich in die Außenumgebung 136 abgegeben wird, gewährleistet.

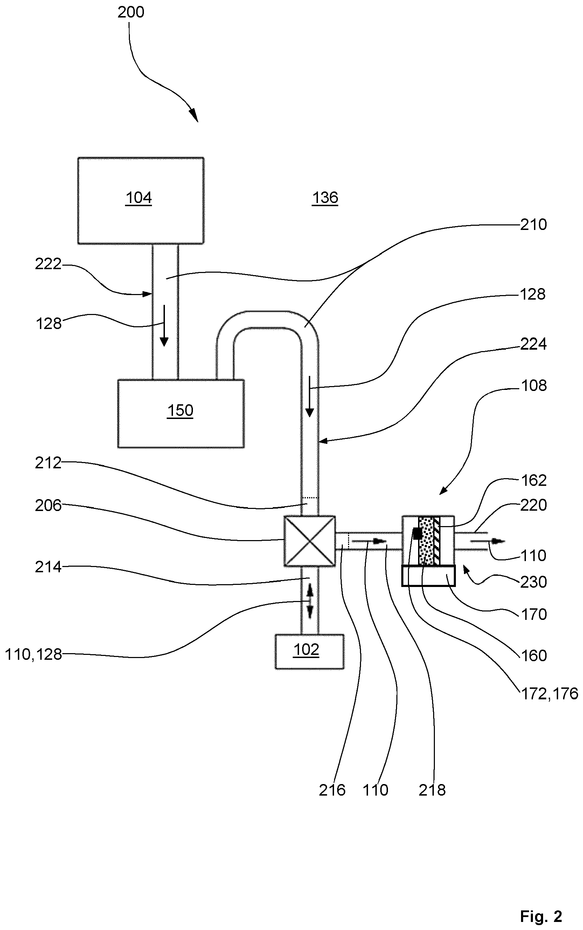

Die Fig. 2 zeigt eine schematische Darstellung einer zweiten Ausführungsform eines Beatmungssystem. Das Beatmungssystem 200 weist wiederum das Beatmungsgerät 104 mit der zugeordneten Filtrationseinrichtung 108 auf. Ein von dem Beatmungsgerät 104 aktiv geregeltes, patientennahes Ausatemventil 206 ist mittels eines Zuführschlauchs 210 mit dem Beatmungsgerät 104 verbunden. In den Zuführschlauch 210 ist wiederum die Konditioniereinrichtung 150 von Fig. 1 eingeschleift, um sicherzustellen, dass die Atemluft 128 eine Temperatur von etwa 39 °C bei einer konstanten relativen Luftfeuchtigkeit von ungefähr 98 % aufweist. Mittels des Zuführschlauchs 210 wird die solchermaßen aufbereitete Einatemluft 128 zu einem Einlassanschluss 212 des Ausatemventils 206 geleitet.

Über einen Beatmungsanschluss 214 des Ausatemventils 206 erfolgt die Beatmung des Patienten 102. Das Ausatemventil 206 verfügt ferner über einen Auslassanschluss 216, über den die ungefilterte Ausatemluft 110 über einen hier nur beispielhaft dargestellten, optionalen Ausatemschlauch 218 bis zur Filtrationseinrichtung 108 von Fig. 1 und von dort bis zu einem Auslass 220 strömt, über den die keimfrei gefilterte Ausatemluft 110 in die Außenumgebung 136 des Beatmungssystems 200 abgegeben wird. Ein erster Abschnitt 222 des Zuführschlauchs 210 zwischen dem Beatmungsgerät 104 und der Konditioniereinrichtung 150 ist unbeheizt, während ein zweiter Abschnitt 224 des Zuführschlauchs 210 zwischen der Konditioniereinrichtung 150 und dem Ausatemventil 206 vorzugsweise beheizbar ausgebildet ist.

Erforderlichenfalls kann der optionale Ausatemschlauch 218 zumindest abschnittsweise beheizbar ausgebildet sein, um eine unerwünschte Kondensation von Feuchtigkeit in der Ausatemluft 110 auch in diesem Bereich zu verhindern. Alternativ kann die Filtrationseinrichtung 108 direkt, das heißt ohne den Ausatemschlauch 218 oder mittels eines nur kurzen Ausatemschlauchs 218 mit dem Ausatemventil 206 verbunden sein. Innerhalb des Beatmungsanschlusses 214 strömt die Einatemluft 128 sowie die Ausatemluft 110 - wie mit dem schwarzen Doppelpfeil angedeutet ist - in zwei entgegengesetzten Richtungen.

Die Filtrationseinrichtung 108 umfasst entsprechend zu der Fig. 1 den Filter 160, die Heizung 162, den mindestens einen Temperatursensor 172, den optionalen Feuchtigkeitssensor 176 und die Regeleinrichtung 170, so dass zwecks Knappheit der Beschreibung und um inhaltliche Dopplungen zu vermeiden, hinsichtlich der weiteren konstruktiven Details hier auf die einschlägigen Beschreibungsabschnitte von Fig. 1 verwiesen sei.

Durch die Filtrationseinrichtung 108 ist eine Belastung der Außenumgebung mit mikrobiellen Keimen und eine hiermit einhergehende Gefährdung Dritter ausgeschlossen, wobei die Heizung 160 der Filtrationseinrichtung 108 die Kondensation von in der Ausatemluft 110 enthaltener Feuchtigkeit stromabwärts hinter dem Filter 160 in einem Kondensationsbereich 230 zuverlässig verhindert.

Ein in den Figuren nicht dargestelltes Beatmungssystem für die so genannte IPV ("Intrapulmonare Perkussive Ventilation") weist einen dem konstruktiven Aufbau des Beatmungssystems 200 von Fig. 2 weitgehend ähnlichen Aufbau auf. Die Filtrationseinrichtung 108 ist in einer derartigen Konstellation jedoch an den offenen Schenkel bzw. an eine definierte Leckage des dem Patienten zugewandten Beatmungsanschlusses 214 angeschlossen, um eine Kontamination der Außenumgebung 136 mit in der Ausatemluft 110 des Patienten 102 enthaltenen mikrobiellen Keimen, wie zum Beispiel Viren, Bakterien, Pilzen oder dergleichen zu verhindern. Unter dem Terminus "Leckage" wird in diesem Zusammenhang eine fluidische Verbindung zwischen dem Beatmungsanschluss des Ausatemstücks und der Außenumgebung verstanden, über die ein begrenzter Luftaustausch mit einem definierten Volumenstrom möglich ist.

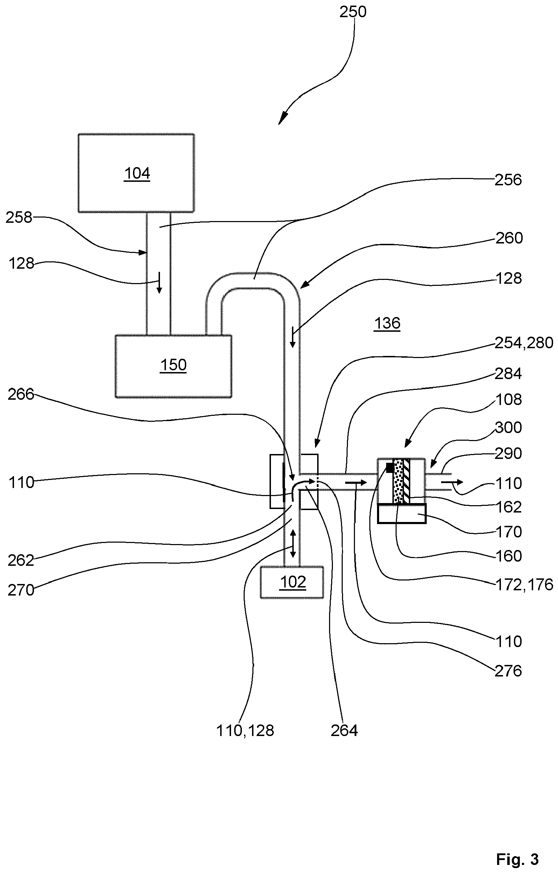

Die Fig. 3 zeigt eine schematische Darstellung einer dritten Ausführungsform eines Beatmungssystems. Das Beatmungssystem 250 umfasst das Beatmungsgerät 104 sowie die Filtrationseinrichtung 108. Ein hier patientennah angeordnetes Ausatemstück 254 ist mittels eines Zuführschlauchs 256 mit dem Beatmungsgerät 104 verbunden. In den Zuführschlauch 256 ist wiederum die Konditioniereinrichtung 150 zur Einstellung von Temperatur und relativer Feuchtigkeit der Atemluft 128 für den Patienten 102 eingeschleift. Ein erster Abschnitt 258 des Zuführschlauchs 256 zwischen dem Beatmungsgerät 104 und der Konditioniereinrichtung 150 ist unbeheizt, während ein zweiter Abschnitt 260 des Zuführschlauchs 256 zwischen der Konditioniereinrichtung 150 und dem Ausatemstück 254 bevorzugt beheizbar ausgebildet ist.

Das Ausatemstück 254 weist eine durchgehende Längsbohrung 262 mit einer ungefähr mittig an dieser ausgebildeten Abzweigbohrung 264 auf. Die Längsbohrung 262 und die Abzweigbohrung 264 verlaufen im Wesentlichen rechtwinklig zueinander und bilden einen Innenraum 266 aus. Der Zuführschlauch 256 ist fluidisch über die Längsbohrung 262 mit einem an das Ausatemstück 254 angeschlossenen Beatmungsschlauch 270 verbunden, über den der Patient 102 beatmet wird.

Bevorzugt im Bereich der rechtwinkligen Abzweigbohrung 264 des Ausatemstücks 254 ist eine Querschnittsreduzierung 276 angelegt, durch die für den Patienten 102 ein definierter Ausatemwiderstand entsteht bzw. geschaffen wird. Diese Querschnittsreduzierung 276 kann beispielsweise mit einer schlitz-, netz-, gewebe-, bohrungsraster- oder siebartigen Struktur gebildet sein. Die Abzweigbohrung 264 ist mit einem Auslassanschluss 284 verbunden, an den wiederum die Filtrationseinrichtung 108 des Beatmungssystems 250 angeschlossen ist. Die den Ausatemwiderstand erhöhende Querschnittsreduzierung 276 kann alternativ auch nur in dem Bereich des Auslassanschlusses 284 vorgesehen sein. Gegebenenfalls kann ergänzend zu der Querschnittsreduzierung 276 innerhalb des Ausatemstücks 254 eine weitere, nicht dargestellte Querschnittsreduzierung in den Auslassanschluss 284 eingebaut sein.

Innerhalb des Beatmungsschlauchs 270 strömen die Einatemluft 128 und die Ausatemluft 110 periodisch dem Atmungsrhythmus des Patienten 102 entsprechend in entgegengesetzten Richtungen, wie mit dem schwarzen Doppelpfeil symbolisiert ist. Innerhalb der Abzweigbohrung 264 bzw. des Auslassanschlusses 284 strömt die Ausatemluft 110 des Patienten 102 im Wesentlichen, das heißt bevorzugterweise nur in Richtung der beheizbaren Filtrationseinrichtung 108 und damit in die Außenumgebung 136. Durch die Querschnittsreduzierung 276 ist sichergestellt, dass die Ausatemluft 110 des Patienten 102 ausgehend von der Filtrationseinrichtung 108 nicht ohne weiteres wieder in Richtung des hier nach Art eines T-Stücks 280 ausgebildeten Ausatemstücks 254 zurückströmen kann.

Die betriebsnotwendigen Drücke der Einatemluft 128 werden von dem Beatmungsgerät 104 durch eine entsprechende Erhöhung eines Volumenstroms bzw. Förderstroms der Einatemluft 128 sichergestellt.

Die Filtrationseinrichtung 108 verfügt zudem über einen rohrartigen Auslass 290, über den die von etwaigen mikrobiellen Kontaminationen mit Hilfe der Filtrationseinrichtung 108 vollständig gereinigte bzw. dekontaminierte Ausatemluft 110 des Patienten 102 in die Außenumgebung 136 des Beatmungssystems 250 geleitet wird.

Die Filtrationseinrichtung 108 umfasst entsprechend zu den Ausführungsformen von Fig. 1, 2 wiederum den Filter 160, die Heizung 162, den mindestens einen Temperatursensor 172, den optional vorgesehenen Feuchtigkeitssensor 176, den unter Umständen vorhandenen Betauungssensor sowie die elektronische Regeleinrichtung 170, so dass hinsichtlich der weiteren konstruktiven Details an dieser Stelle insbesondere auf die relevanten Teile der Beschreibung von Fig. 1 verwiesen sei. Der Auslassanschluss 284 kann gleichfalls zumindest abschnittsweise beheizbar ausgebildet sein.

Durch die Filtrationseinrichtung 108 wird eine Belastung bzw. Kontamination der Außenumgebung 136 mit insbesondere krankmachenden mikrobiellen Keimen, wie Viren, Bakterien, Pilzen etc. in der Ausatemluft 110 des Patienten zuverlässig vermieden. Durch die dem Filter 160 zugeordnete Heizung 162 der Filtrationseinrichtung 108 wird darüber hinaus eine Kondensation der in der Regel feuchten Ausatemluft 110 des Patienten 102 stromabwärts hinter dem Filter 160 der Filtrationseinrichtung 108 verhindert. Durch das Unterbinden der Kondensation der in der Ausatemluft 110 des Patienten 102 enthaltenen Feuchtigkeit ist eine Verkeimung eines stromabwärts hinter der Filtrationseinrichtung 108 befindlichen Kondensationsbereichs 300 mit nahezu vollständiger Sicherheit ausgeschlossen.

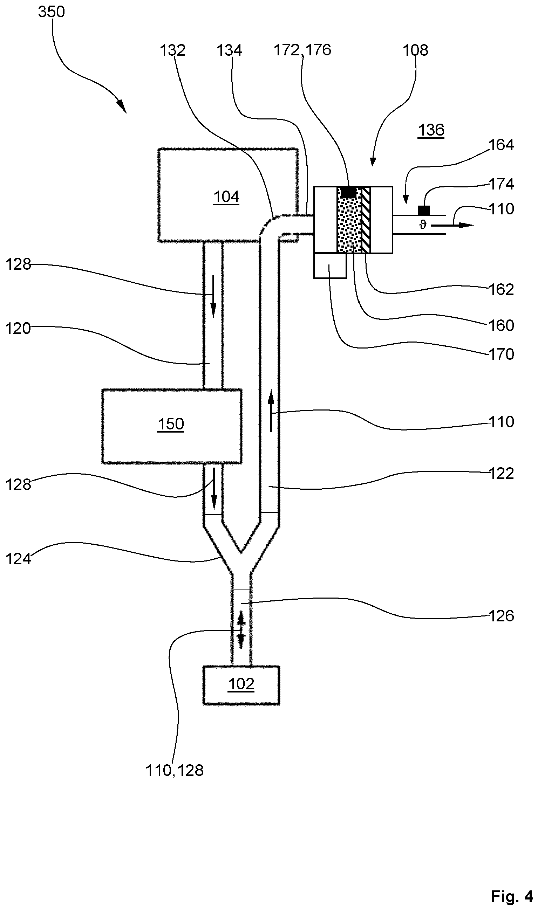

Die Fig. 4 zeigt eine schematische Darstellung einer vierten Ausführungsform eines Beatmungssystems. Die vierte Ausführungsform eines Beatmungssystems 350 zur Verbesserung der Beatmung eines Patienten 102 umfasst erneut das Beatmungsgerät 104 mit der Filtrationseinrichtung 108 zur Reduzierung der Keimbelastung der Ausatemluft 110 des Patienten. Das Beatmungsgerät 104 des Beatmungssystems 100 verfügt über den Einatemschlauch 120 sowie den Ausatemschlauch 122, die mittels des Y-Verbindungsstücks 124 zu dem Beatmungsschlauch 126 zur Beatmung des Patienten 102 zusammengeführt sind. Mittels des Einatemschlauchs 120 und dem Y-Verbindungsstück 124 mit dem nachfolgenden Beatmungsschlauch 126 wird dem Patienten 102 von dem Beatmungsgerät 104 bereitgestellte Einatemluft 128 zugeführt und über den Ausatemschlauch 122 als mit Keimen des Patienten belastete Ausatemluft 110 wieder in Richtung des Beatmungsgeräts 104 abgeführt. In den Einatemschlauch 120 ist wiederum die Konditioniereinrichtung 150 eingeschleift. Innerhalb des Beatmungsschlauchs 126 strömen die Einatemluft 128 und die Ausatemluft 110 in entgegengesetzten Richtungen, was mit einem schwarzen Doppelpfeil veranschaulicht ist.
Der stromabwärts liegende Bogen 132 des Ausatemschlauchs 122 führt exemplarisch durch das Beatmungsgerät 104 hindurch bis zu dem Auslass 134, der - abweichend von der ersten Ausführungsform nach Fig. 1 - in die stromabwärts nachgeschaltete Filtrationseinrichtung 108 führt. Infolgedessen durchströmt die Ausatemluft 110 des Patienten 102 abweichend von der ersten Ausführungsform nach Maßgabe von Fig. 1 hier zunächst das Beatmungsgerät 104 und erst anschließend die Filtrationseinrichtung 108. Nach dem Durchströmen der Filtrationseinrichtung 108 und des sich daran anschließenden Kondensationsbereichs 164 des Ausatemschlauchs 122 gelangt die dekontaminierte Ausatemluft 110 in die Außenumgebung 136 des Beatmungssystems 350.

Die Filtrationseinrichtung 108 des Beatmungssystems 350 verfügt erneut über die elektronische Regeleinrichtung 170, mittels der die Temperatur ϑ der Ausatemluft 110 stromabwärts bzw. ausgangsseitig des Filters 160 so regelbar ist, dass eine Kondensation der feuchtigkeitsbeladenen Ausatemluft 110 innerhalb des Kondensationsbereichs 164 des Ausatemschlauchs 122 vermieden wird. Zu diesem Zweck wirkt die Regeleinrichtung 170 mit dem Temperatursensor 172 zusammen, der zum Beispiel im Bereich des Filters 160 und/oder der Heizung 162 der Filtrationseinrichtung 108 platziert sein kann. Mindestens ein weiterer Temperatursensor 174 kann weiter stromabwärts hinter der Filtrationseinrichtung 108 im Kondensationsbereich 164 vorgesehen sein. Erforderlichenfalls kann zusätzlich mindestens ein Feuchtigkeitssensor 176 und/oder mindestens ein Betauungssensor im Bereich des Filters 160 und/oder der Heizung 162 der Filtrationseinrichtung 108 platziert sein.

Im Übrigen folgt der Aufbau der vierten Ausführungsform des Beatmungssystems 350 dem der ersten Ausführungsform des Beatmungssystems von Fig. 1, so dass an dieser Stelle zwecks Knappheit und Kürze der Beschreibung sowie zur Vermeidung inhaltlicher Doppelungen auf die einschlägigen Beschreibungsteile von Fig. 1 verwiesen sei.

Die Erfindung betrifft ein Beatmungssystem 100, 200, 250, 350 zur Verbesserung der Beatmung eines Patienten 102, mit einem Beatmungsgerät 104 und mit einer Filtrationseinrichtung 108 zur Reduzierung einer Keimbelastung von Ausatemluft 110 des Patienten 102. Die Filtrationseinrichtung 108 weist einen Filter 160 und eine Heizung 162 zum Beheizen des Filters 160 auf, wobei die Erwärmung des Filters 160 derart erfolgt, dass ein Kondensationsbereich 164, 230, 300 der Ausatemluft 110 stromabwärts und außerhalb des Filters 160 liegt. Durch die Filtrationseinrichtung 108 des Beatmungssystems 100, 200, 250, 350 wird eine Kontamination der Außenumgebung 136 mit Keimen des Patienten 102, wie beispielsweise Viren, Bakterien, Pilzen, zuverlässig vermieden.

### Bezugszeichenliste

- 100: Beatmungssystem (1. Var.)
- 102: Patient
- 104: Beatmungsgerät
- 108: Filtrationseinrichtung
- 110: Ausatemluft (Patient)
- 120: Einatemschlauch
- 122: Ausatemschlauch
- 124: Y-Verbindungsstück
- 126: Beatmungsschlauch
- 128: Einatemluft
- 132: Bogen
- 134: Auslass (Außenumgebung)
- 136: Außenumgebung
- 150: Konditioniereinrichtung
- 160: Filter
- 162: Heizung
- 164: Kondensationsbereich
- 170: Regeleinrichtung (Filtrationseinrichtung)
- 172: Temperatursensor
- 174: Temperatursensor
- 176: Feuchtigkeitssensor
- 200: Beatmungssystem (2. Var.)
- 206: Ausatemventil
- 210: Zuführschlauch
- 212: Einlassanschluss (Beatmungsventil)
- 214: Beatmungsanschluss (Beatmungsventil)
- 216: Auslassanschluss (Beatmungsventil)
- 218: Ausatemschlauch
- 220: Auslass (Außenumgebung)
- 222: erster Abschnitt (Zuführschlauch)
- 224: zweiter Abschnitt (Zuführschlauch)
- 230: Kondensationsbereich
- 250: Beatmungssystem (3. Var.)
- 254: Ausatemstück
- 256: Zuführschlauch
- 258: erster Abschnitt (Zuführschlauch)
- 260: zweiter Abschnitt (Zuführschlauch)
- 262: durchgehende Längsbohrung
- 264: Abzweigbohrung
- 266: Innenraum
- 270: Beatmungsanschluss (Ausatemstück)
- 276: Querschnittsreduzierung
- 280: T-Stück
- 284: Auslassanschluss (Ausatemstück)
- 290: Auslass (Filtrationseinrichtung)
- 296: schwarzer Pfeil
- 300: Kondensationsbereich
- 350: Beatmungssystem (4. Var.)
- ϑ: Temperatur (Ausatemluft)

## Patentansprüche

1. Beatmungssystem (100, 200, 250, 350) zur Verbesserung der Beatmung eines Patienten (102), mit einem Beatmungsgerät (104) und mit einer Filtrationseinrichtung (108) zur Reduzierung einer Keimbelastung von Ausatemluft (110) des Patienten (102), **dadurch gekennzeichnet, dass** die Filtrationseinrichtung (108) einen Filter (160) und eine Heizung (162) zum Beheizen des Filters (160) aufweist, wobei die Erwärmung des Filters (160) derart erfolgt, dass ein Kondensationsbereich (164, 230, 300) der Ausatemluft (110) stromabwärts und außerhalb des Filters (160) liegt.

2. Beatmungssystem (100, 200, 250, 350) nach Patentanspruch 1, **dadurch gekennzeichnet, dass** die Filtrationseinrichtung (108) eine Regeleinrichtung (170) zum Regeln einer Temperatur (ϑ) stromabwärts des Filters (160) aufweist.

3. Beatmungssystem (100, 350) nach Patentanspruch 1 oder 2, **dadurch gekennzeichnet, dass** das Beatmungsgerät (104) einen Einatemschlauch (120) und einen Ausatemschlauch (122) aufweist, die mittels eines Y-Verbindungsstücks (124) zu einem Beatmungsschlauch (126) zur Beatmung des Patienten (102) zusammengeführt sind, wobei die Filtrationseinrichtung (108) patientenfern und außerhalb des Beatmungsgeräts (104) in dem Ausatemschlauch (122) angeordnet ist.

4. Beatmungssystem (100, 350) nach Patentanspruch 3, **dadurch gekennzeichnet, dass** der Einatemschlauch (120) eine Konditioniereinrichtung (150) aufweist.

5. Beatmungssystem (100, 350) nach Patentanspruch 3 oder 4, **dadurch gekennzeichnet, dass** der Ausatemschlauch (122) zumindest abschnittsweise beheizbar ist.

6. Beatmungssystem (100) nach Patentanspruch 3, 4 oder 5, **dadurch gekennzeichnet, dass** die Ausatemluft (110) des Patienten (102) zunächst durch die Filtrationseinrichtung (108) und dann durch das Beatmungsgerät (108) führbar ist.

7. Beatmungssystem (350) nach Patentanspruch 3, 4 oder 5, **dadurch gekennzeichnet, dass** die Ausatemluft (100) des Patienten (102) zunächst durch das Beatmungsgerät (108) und dann durch die Filtrationseinrichtung (108) führbar ist.

8. Beatmungssystem (200) nach Patentanspruch 1 oder 2, **dadurch gekennzeichnet, dass** das Beatmungsgerät (104) mittels eines Zuführschlauchs (210) mit einem patientennahen Ausatemventil (206) verbunden ist, wobei ein Auslassanschluss (216) des Ausatemventils (206) direkt oder mittels eines vorzugsweise beheizbaren Ausatemschlauchs (218) mit der Filtrationseinrichtung (108) verbunden ist und der Patient (102) über einen Beatmungsanschluss (214) des Ausatemventils (206) beatembar ist.

9. Beatmungssystem (250) nach Patentanspruch 1 oder 2, **dadurch gekennzeichnet, dass** das Beatmungsgerät (104) mittels eines Zuführschlauchs (256) mit einem patientennahen Ausatemstück (254) verbunden ist, wobei ein Auslassanschluss (284) des Ausatemstücks (254) mit der Filtrationseinrichtung (108) verbunden ist und der Patient (102) über einen Beatmungsanschluss (270) des Ausatemstücks (254) beatembar ist.

10. Beatmungssystem (250) nach Patentanspruch 9, **dadurch gekennzeichnet, dass** das Ausatemstück (254) und/oder der Auslassanschluss (284) eine Querschnittsreduzierung (276) aufweist, derart, dass beim Ausatmen des Patienten (102) ein Ausatemwiderstand entsteht durch den die Ausatemluft (110) bevorzugt zu der Filtrationseinrichtung (108) geleitet wird.

11. Beatmungssystem (250) nach Patentanspruch 8, 9 oder 10, **dadurch gekennzeichnet, dass** der Zuführschlauch (210, 256) eine Konditioniereinrichtung (150) aufweist.

12. Beatmungssystem (100, 200, 250, 350) nach einem der vorhergehenden Patentansprüche, **dadurch gekennzeichnet, dass** die Heizung (162) des Filters (160) der Filtrationseinrichtung (108) mit mindestens einer beheizbaren Kontaktfläche, mindestens einem, den Filter (160) zumindest bereichsweise umschließenden Heizkörper, einem Infrarotstrahler, einem elektrischen Widerstandsheizelement oder dergleichen gebildet ist und der Regeleinrichtung (170) mindestens ein bevorzugt im Bereich des Filters (160) angeordneter Temperatursensor (172) zugeordnet ist.
